# EUROPEAN PATENT APPLICATION

(11) **EP 4 492 397 A1**
(43) Date of publication of application: **15.01.2025**
(21) Application number: 23184382.2
(22) Date of filing: 10.07.2023
(51) Int. Cl.: G16H 40/67, A61N 1/372, H04W 12/06, G16H 80/00

(54) **METHOD AND SYSTEM FOR AUTHENTICATING AN IMPLANTABLE MEDICAL DEVICE**

(71) Applicant: BIOTRONIK SE & Co. KG, 12359 Berlin (DE)
(72) Inventor: Krüger, Daniel, 15741 Bestensee (DE); Müller, Jens, 14195 Berlin (DE); Deutschmann, Hendrik, 10247 Berlin (DE)
(74) Representative: Biotronik Corporate Services SE

(57) **Abstract**

The invention relates to a computer-implemented method and system for authenticating an implantable medical device (10) during a registration process for activating access of the implantable medical device (10) to a remote service (14), comprising the steps of providing (S1) registration data (16) through a web-interface (18a) and/or a patient app (18b) on a patient communication device (12), said registration data (16) comprising at least one of a username, a password and a serial number of the implantable medical device (10), in reaction to receiving the registration data (16), sending (S2) a verification request (R) to the web-interface (18a) and/or a patient app (18b) on the patient communication device (12), and verifying (S3) the registration of the implantable medical device (10) for the remote service (14) by triggering the implantable medical device (10) to send an authentication code (C) or to send a data set (DS) comprising a timestamp and the serial number of the implantable medical device (10).

## Description

The invention relates to a computer-implemented method for authenticating an implantable medical device during a registration process for activating access of the patient communication device to a remote service.

Furthermore, the invention relates to a system for authenticating an implantable medical device during a registration process for activating access of the patient communication device to a remote service.

Currently, registration of implantable medical devices and/or patient communication devices communicating with respective implantable medical devices for remote services is carried out through medical staff during implantation or follow up sessions.

Such remote services can e.g. provide the patient with medical data, analysis, statistics and/or history of medical data captured by the implantable medical device. Furthermore, the patient can provide status reports on his or her wellbeing through a patient app to the remote service.

There is hence currently no means for the patient to register for a remote service independently of medical staff. Therefore, there is a need for a more efficient method for registering an implantable medical device and/or a patient communication device communicating with a respective implantable medical device for a remote service.

It is therefore an object of the present invention to provide an improved registration process for providing access of the patient communication device communicating with the respective implantable medical device to a remote service.

The object is solved by a computer-implemented method for authenticating an implantable medical device during a registration process for activating access of the patient communication device to a remote service having the features of claim 1.

Furthermore, the object is solved by a system for authenticating an implantable medical device during a registration process for activating access of the patient communication device to a remote service having the features of claim 14.

In addition, the object is solved by computer program having the features of claim 15.

Further developments and advantageous embodiments are defined in the dependent claims.

The present invention provides a computer-implemented method for authenticating an implantable medical device during a registration process for activating access of the patient communication device to a remote service.

The method comprises providing registration data through a web-interface and/or a patient app, in particular a remote service frontend, on the patient communication device, said registration data comprising at least one of a username, a password and a serial number of the implantable medical device.

Furthermore, the method comprises in reaction to receiving the registration data, sending a verification request to the web-interface and/or the patient app on the patient communication device by the remote service backend.

The method moreover comprises verifying a registration of the patient communication device for the remote service by triggering the implantable medical device to send an authentication code or to send a data set comprising a timestamp and the serial number of the implantable medical device to an authentication module.

In addition, the present invention provides a system for authenticating an implantable medical device during a registration process for activating access of the patient communication device to a remote service.

The system comprises a web-interface and/or a patient app, in particular a remote service frontend, on the patient communication device configured to provide registration data, said registration data comprising at least one of a username, a password and a serial number of the implantable medical device.

Furthermore, the system comprises the remote service backend for sending a verification request to the web-interface and/or the patient app on the patient communication device in reaction to receiving the registration data.

The system moreover comprises the implantable medical device being configured to be triggered to send an authentication code or to send a data set comprising a timestamp and the serial number of the implantable medical device to an authentication module to verify a registration of the patient communication device for the remote service.

An idea of the present invention is to provide digital remote B2C (business to consumer) services for patients with implants without involving medical staff in the registration process for these services. This self-managed registration is a fundamental property of B2C services and opens the possibility to provide this kind of services. Furthermore, this eases the workflows for clinics and helps to reduce costs by minimizing support of medical staff for these services.

Furthermore, a secure registration process is provided by using a two-factor authentication, the first factor comprising at least one of a username, a password and a serial number of the implantable medical device and the second factor comprising sending an authentication code or sending a data set comprising a timestamp and the serial number of the implantable medical device to an authentication module.

According to an aspect of the invention, the registration data is provided through the web-interface and/or the patient app on a patient communication device, in particular a smartphone or a tablet computing device, to the authentication module and/or the remote service backend, wherein the authentication module and the remote service backend are hosted on at least one server of an IT service provider or on at least one server of a health service provider.

The patient is thus able to conveniently use the web-interface and/or the patient app on his or her patient communication device in order to register for a desired remote service.

Said remote service can e.g. provide the patient with information about the current status of the implant, e.g. a battery status. The service can further allow the patient to report his or her well-being to the healthcare provider.

The physician then, in addition to the measurement data of the implant, has another source of data available to determine whether or not a programming of the implantable medical device needs to be changed. A live coaching function is also conceivable, in which the remote service analyzes the information provided by the patient and gives feedback/recommendations for an appropriate action that the patient can take to improve reported symptoms.

According to a further aspect of the invention, upon providing the registration data, a registration confirmation request is received by the web-interface and/or the patient app on the patient communication device from the authentication module and/or from the remote service backend.

The registration confirmation request advantageously enables a two-factor authentication to be performed, thus securely identifying the implantable medical device linked to the patient communication device.

According to a further aspect of the invention, upon receiving the registration confirmation request, a registration confirmation is sent through the web-interface and/or the patient app on the patient communication device to the authentication module and/or to the remote service backend. By sending the registration confirmation, the patient thus confirms that the first authentication step is completed.

According to a further aspect of the invention, upon receipt of the registration confirmation, the remote service backend and/or the authentication module sends a verification request to the web-interface and/or the patient app on the patient communication device. By receiving the verification request, the patient is thus informed to initiate a procedure to perform authentication by means of the second authentication factor.

According to a further aspect of the invention, the verification request comprises a prompt to the patient to trigger the implantable medical device to send the authentication code or to send the data set comprising a timestamp and the serial number of the implantable medical device to the authentication module and/or to the remote service backend. This step can advantageously be performed by the patient in order to cause the implantable medical device to communicate the authentication code or the data set comprising the timestamp and the serial number of the implantable medical device to the authentication module and/or to the remote service backend.

According to a further aspect of the invention, the authentication module sends a control command to the implantable medical device to set the implantable medical device in a verification mode. The implantable medical device is thus ready to be triggered by a trigger device.

According to a further aspect of the invention, the implantable medical device is triggered to send the authentication code or to send the data set comprising the timestamp and the serial number of the implantable medical device by receiving a trigger communication by a trigger device.

Only the patient having the implantable medical device is thus in a position to trigger the implantable medical device to send the authentication code or to send the data set comprising the timestamp and the serial number of the implantable medical device to the authentication module and/or to the remote service backend which poses a security advantage.

According to a further aspect of the invention, the trigger device is a magnet or a magnetic coil configured to apply a magnetic field to the implantable medical device or wherein the trigger device is the patient communication device configured to communicate with the implantable medical device via MICS-band or Bluetooth low energy communication. The trigger device is hence a device that is at the patient's disposal all the time.

According to a further aspect of the invention, in reaction to receiving the control command and being triggered by the trigger device, the implantable medical device sends the authentication code or the data set comprising the timestamp and the serial number of the implantable medical device via MICS-band or Bluetooth low energy communication to the patient communication device.

The implantable medical device thus uniquely identifies itself. The identification data is furthermore immutably stored in a data storage of the implantable medical device, wherein said identification data is saved in the implantable medical device during production of the implantable medical device.

According to a further aspect of the invention, the patient communication device forwards the received authentication code or the data set comprising the timestamp and the serial number of the implantable medical device to the authentication module. Said authentication module, based on the received data, is thus capable of determining whether or not the patient is qualified and/or entitled to request access to said remote service.

According to a further aspect of the invention, upon receipt of the authentication code or the data set comprising the timestamp and the serial number of the implantable medical device, the authentication module and/or the remote service backend compares the authentication code to a stored authentication code associated with the implantable medical device or wherein the authentication module and/or the remote service backend compares, if the timestamp associated with the serial number of the implantable medical device is received within a predetermined time period. Said comparison advantageously enables a secure authentication of the patient as the authentication data is only known to the manufacturer of the implantable medical device.

According to a further aspect of the invention, if the authentication code sent by the implantable medical device matches the authentication code stored in a data storage of the authentication module and/or the remote service backend of if the timestamp associated with the serial number of the implantable medical device is received within the predetermined time period, the authentication module releases the remote service, in particular activates access to the remote service on the patient communication device.

The above-mentioned procedure thus advantageously puts the patient in a position to request access the desired remote service without the need to involve a healthcare practitioner and/or an IT service provider.

The herein described features of the computer-implemented method for authenticating an implantable medical device during a registration process for activating access of the patient communication device to a remote service also disclosed for the system for authenticating an implantable medical device during a registration process for activating access of the patient communication device to a remote service and vice versa.

For a more complete understanding of the present invention and advantages thereof, reference is now made to the following description taken in conjunction with the accompanying drawings. The invention is explained in more detail below using exemplary embodiments, which are specified in the schematic figures of the drawings, in which:
- Fig. 1: shows a flowchart of a computer-implemented method for authenticating an implantable medical device during a registration process for activating access of the patient communication device to a remote service according to a preferred embodiment of the invention; and
- Fig. 2: shows a diagram of a system for authenticating an implantable medical device during a registration process for activating access of the patient communication device to a remote service according to the preferred embodiment of the invention.

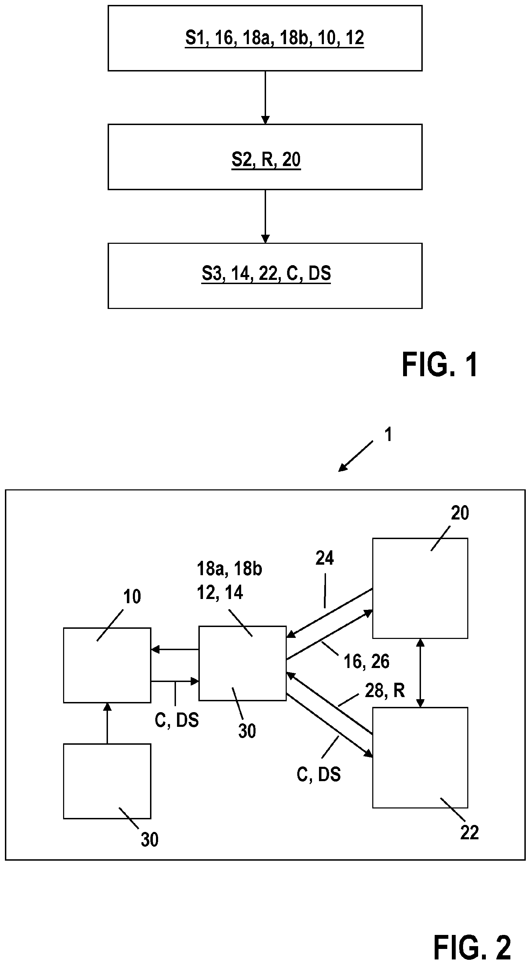

The computer-implemented method for authenticating an implantable medical device 10 during a registration process for activating access of the patient communication device 12 to a remote service 14 shown in Fig. 1 comprises providing S1 registration data 16 through a web-interface 18a and/or a patient app 18b, in particular a remote service frontend, on the patient communication device 12, said registration data 16 comprising at least one of a username, a password and a serial number of the implantable medical device 10.

Furthermore, the method comprises in reaction to receiving the registration data 16, sending S2 a verification request R to the web-interface 18a and/or the patient app 18b on the patient communication device 12 by the remote service backend 20.

Moreover, the method comprises verifying S3 a registration of the patient communication device 12 for the remote service 14 by triggering the implantable medical device 10 to send an authentication code C or to send a data set DS comprising a timestamp and the serial number of the implantable medical device 10 to an authentication module 22.

The registration data 16 is provided through the web-interface 18a and/or the patient app 18b on a patient communication device 12, in particular a smartphone or a tablet computing device, to the authentication module 22 and/or the remote service backend 20, wherein the authentication module 22 and the remote service backend 20 are hosted on at least one server of an IT service provider or on at least one server of a health service provider.

In addition, upon providing the registration data 16, a registration confirmation request 24 is received by the web-interface 18a and/or the patient app 18b on the patient communication device 12 from the authentication module 22 and/or from the remote service backend 20. Upon receiving the registration confirmation request 24, a registration confirmation 26 is sent through the web-interface 18a and/or the patient app 18b on the patient communication device 12 to the authentication module 22 and/or to the remote service backend 20.

Furthermore, upon receipt of the registration confirmation 26, the remote service backend 20 and/or the authentication module 22 sends a verification request R to the web-interface 18a and/or the patient app 18b on the patient communication device 12. The verification request R comprises a prompt to the patient to trigger the implantable medical device 10 to send the authentication code C or to send the data set DS comprising a timestamp and the serial number of the implantable medical device 10 to the authentication module 22 and/or to the remote service backend 20.

Moreover, the authentication module 22 sends a control command 28 to the implantable medical device 10 to set the implantable medical device 10 in a verification mode. The implantable medical device 10 is then triggered to send the authentication code C or to send the data set DS comprising the timestamp and the serial number of the implantable medical device 10 by receiving a trigger communication by a trigger device 30.

The trigger device 30 is a magnet or a magnetic coil configured to apply a magnetic field to the implantable medical device 10. Alternatively, the trigger device 30 is the patient communication device 12 configured to communicate with the implantable medical device 10 via MICS-band or Bluetooth low energy communication.

In reaction to receiving the control command 28 and being triggered by the trigger device 30, the implantable medical device 10 sends the authentication code C or the data set DS comprising the timestamp and the serial number of the implantable medical device 10 via MICS-band or Bluetooth low energy communication to the patient communication device 12.

Furthermore, the patient communication device 12 forwards the received authentication code C or the data set DS comprising the timestamp and the serial number of the implantable medical device 10 to the authentication module 22.

Upon receipt of the authentication code C or the data set DS comprising the timestamp and the serial number of the implantable medical device 10, the authentication module 22 and/or the remote service backend 20 compares the authentication code C to a stored authentication code C associated with the implantable medical device 10. Alternatively, the authentication module 22 and/or the remote service backend 20 compares, if the timestamp associated with the serial number of the implantable medical device 10 is received within a predetermined time period.

If the authentication code C sent by the implantable medical device 10 matches the authentication code C stored in a data storage of the authentication module 22 and/or the remote service backend 20 of if the timestamp associated with the serial number of the implantable medical device 10 is received within the predetermined time period, the authentication module 22 releases the remote service 14, in particular activates access to the remote service 14 on the patient communication device 12.

Fig. 2 shows a diagram of a system 1 for authenticating an implantable medical device 10 during a registration process for activating access of the patient communication device 12 to a remote service 14 according to the preferred embodiment of the invention.

The system 1 comprises a web-interface 18a and/or a patient app 18b, in particular a remote service frontend, on the patient communication device 12 configured to provide registration data 16, said registration data 16 comprising at least one of a username, a password and a serial number of the implantable medical device 10.

Moreover, the system 1 comprises the remote service backend 20 for sending a verification request R to the web-interface 18a and/or the patient app 18b on the patient communication device 12 in reaction to receiving the registration data 16.

The system 1 furthermore comprises the implantable medical device 10 being configured to be triggered to send an authentication code C or to send a data set DS comprising a timestamp and the serial number of the implantable medical device 10 to an authentication module 22 to verify a registration of the patient communication device 12 for the remote service 14.

### Reference Signs

- 1: system
- 10: implantable medical device
- 12: patient communication device
- 14: remote service
- 16: registration data
- 18a: web-interface
- 18b: patient app
- 20: remote service backend
- 22: authentication module
- 24: registration confirmation request
- 26: registration confirmation
- 28: control command
- 30: trigger device
- C: authentication code
- DS: data set
- R: verification request
- S1 - S3: method steps

## Claims

1. Computer-implemented method for authenticating an implantable medical device (10) during a registration process for activating access of a patient communication device (12) to a remote service (14), comprising the steps of:
providing (S1) registration data (16) through a web-interface (18a) and/or a patient app (18b), in particular a remote service frontend, on the patient communication device (12), said registration data (16) comprising at least one of a username, a password and a serial number of the implantable medical device (10);
in reaction to receiving the registration data (16), sending (S2) a verification request (R) to the web-interface (18a) and/or the patient app (18b) on the patient communication device (12) by the remote service backend (20); and
verifying (S3) a registration of the patient communication device (12) for the remote service (14) by triggering the implantable medical device (10) to send an authentication code (C) or to send a data set (DS) comprising a timestamp and the serial number of the implantable medical device (10) to an authentication module (22).

2. Computer-implemented method of claim 1, wherein the registration data (16) is provided through the web-interface (18a) and/or the patient app (18b) on a patient communication device (12), in particular a smartphone or a tablet computing device, to the authentication module (22) and/or the remote service backend (20), wherein the authentication module (22) and the remote service backend (20) are hosted on at least one server of an IT service provider or on at least one server of a health service provider.

3. Computer-implemented method of claim 2, wherein upon providing the registration data (16), a registration confirmation request (24) is received by the web-interface (18a) and/or the patient app (18b) on the patient communication device (12) from the authentication module (22) and/or from the remote service backend (20).

4. Computer-implemented method of claim 3, wherein upon receiving the registration confirmation request (24), a registration confirmation (26) is sent through the web-interface (18a) and/or the patient app (18b) on the patient communication device (12) to the authentication module (22) and/or to the remote service backend (20).

5. Computer-implemented method of claim 3 or 4, wherein upon receipt of the registration confirmation (26), the remote service backend (20) and/or the authentication module (22) sends a verification request (R) to the web-interface (18a) and/or the patient app (18b) on the patient communication device (12).

6. Computer-implemented method of claim 5, wherein the verification request (R) comprises a prompt to the patient to trigger the implantable medical device (10) to send the authentication code (C) or to send the data set (DS) comprising a timestamp and the serial number of the implantable medical device (10) to the authentication module (22) and/or to the remote service backend (20).

7. Computer-implemented method of any one of claims 2 bis 6, wherein the authentication module (22) sends a control command (28) to the implantable medical device (10) to set the implantable medical device (10) in a verification mode.

8. Computer-implemented method of claim 7, wherein the implantable medical device (10) is triggered to send the authentication code (C) or to send the data set (DS) comprising the timestamp and the serial number of the implantable medical device (10) by receiving a trigger communication by a trigger device (30).

9. Computer-implemented method of claim 8, wherein the trigger device (30) is a magnet or a magnetic coil configured to apply a magnetic field to the implantable medical device (10) or wherein the trigger device (30) is the patient communication device (12) configured to communicate with the implantable medical device (10) via MICS-band or Bluetooth low energy communication.

10. Computer-implemented method of claim 8 or 9, wherein in reaction to receiving the control command (28) and being triggered by the trigger device (30), the implantable medical device (10) sends the authentication code (C) or the data set (DS) comprising the timestamp and the serial number of the implantable medical device (10) via MICS-band or Bluetooth low energy communication to the patient communication device (12).

11. Computer-implemented method of claim 10, wherein the patient communication device (12) forwards the received authentication code (C) or the data set (DS) comprising the timestamp and the serial number of the implantable medical device (10) to the authentication module (22).

12. Computer-implemented method of claim 2 to 11, wherein upon receipt of the authentication code (C) or the data set (DS) comprising the timestamp and the serial number of the implantable medical device (10), the authentication module (22) and/or the remote service backend (20) compares the authentication code (C) to a stored authentication code (C) associated with the implantable medical device (10) or wherein the authentication module (22) and/or the remote service backend (20) compares, if the timestamp associated with the serial number of the implantable medical device (10) is received within a predetermined time period.

13. Computer-implemented method of claim 12, wherein if the authentication code (C) sent by the implantable medical device (10) matches the authentication code (C) stored in a data storage of the authentication module (22) and/or the remote service backend (20) of if the timestamp associated with the serial number of the implantable medical device (10) is received within the predetermined time period, the authentication module (22) releases the remote service (14), in particular activates access to the remote service (14) on the patient communication device (12).

14. System (1) for authenticating an implantable medical device (10) during a registration process for activating access of the patient communication device (12) to a remote service (14), comprising:
a web-interface (18a) and/or a patient app (18b), in particular a remote service frontend, on the patient communication device (12) configured to provide registration data (16), said registration data (16) comprising at least one of a username, a password and a serial number of the implantable medical device (10);
the remote service backend (20) for sending a verification request (R) to the web-interface (18a) and/or the patient app (18b) on the patient communication device (12) in reaction to receiving the registration data (16); and
the implantable medical device (10) being configured to be triggered to send an authentication code (C) or to send a data set (DS) comprising a timestamp and the serial number of the implantable medical device (10) to an authentication module (22) to verify a registration of the patient communication device (12) for the remote service (14).

15. Computer program with program code to perform the method of any one of claims 1 to 13 when the computer program is executed on a computer.
